# EUROPEAN PATENT APPLICATION

(11) **EP 3 473 729 A1**
(43) Date of publication of application: **24.04.2019**
(21) Application number: 17815395.3
(22) Date of filing: 20.06.2017
(51) Int. Cl.: C12Q 1/68, C12N 15/09

(54) **MMP1 GENE TRANSCRIPT FOR USE AS MARKER FOR DIAGNOSIS OF OVARIAN CANCER PROGNOSIS, AND TEST METHOD**

(30) Priority: 20.06.2016 JP 2016121392
(71) Applicant: National Cancer Center, Tokyo 104-0045 (JP); Biomimetics Sympathies Inc., Tokyo 135-0064 (JP)
(72) Inventor: OCHIYA, Takahiro, Tokyo 104-0045 (JP); YOKOI, Akira, Tokyo 104-0045 (JP); KATO, Tomoyasu, Tokyo 104-0045 (JP)
(74) Representative: Greiner, Elisabeth
(86) International application number: PCT/JP2017/022680
(87) International publication number: WO 2017/221927

(57) **Abstract**

[Problem]To provide: a marker and a kit both for use in the prediction of ovarian cancer prognosis; and a prognosis prediction method.[Solution]A marker and a kit, both for use in the prediction of ovarian cancer prognosis, each of said marker and kit containing a transcript of matrix metalloprotease (MMP) 1 gene; and a method for predicting ovarian cancer prognosis using the marker or the kit, in which the expression level of matrix metalloprotease (MMP) 1 gene in a biological sample collected from a subject is by detecting an ovarian cancer prognosis prediction marker comprising a transcript of matrix metalloprotease (MMP) 1gene and then obtaining a detection value of the ovarian cancer prognosis prediction marker.

## Description

### FIELD OF THE INVENTION

The present invention relates to a marker for detecting a poor prognosis patient group from among patients suffering from ovarian cancer and a detection method thereof.

### BACKGROUND OF THE INVENTION

Ovarian cancer is the worst prognostic cancer type among gynecologic malignant tumors. Its morbidity and mortality rate are steadily rising. In 2010, about 160,000 people worldwide die from ovarian cancer. Ovarian cancer cells are known to have a high affinity for mesothelial cells, the main constituent cells of the peritoneum, and are extremely prone to peritoneal metastasis which is one of important prognostic factors. Drug therapy for peritoneal metastasis is not yet established, and its molecular mechanisms are hardly elucidated. According to the statistical data of 2014, the cumulative risk of females is about 46% (1 per 2 people with cancer) among all cancers, of which about 1% of ovarian cancer (1 in 87 with cancer). In addition, women's cumulative mortality risk is about 16% in all cancer types (1 in 6 people died of cancer), whereas ovarian cancer is about 0.5% (1 in 188 people with cancer death). Thus, ovarian cancer is classified as a cancer with a poor prognosis after morbidity, and the importance of selecting an appropriate treatment combining the characteristics detected in early ovarian cancer has been recognized. As one of poor prognosis, ovarian cancer is often found to have poor subjective symptoms in the early stage, and is discovered at the stage of advanced clinical stage. As another cause, ovarian cancer progresses faster, and it is easy to spread to distant metastasis and lymph nodes even at a small tumor stage, thus it is a tumor with high malignancy.

In order to solve the former, effective markers of early diagnosis need to be established. Currently, after being found by internal examination or echo examination, benignity and malignancy are determined by image diagnosis or measurement of blood tumor marker. Representative diagnostic markers for ovarian cancer are used including CA125 and the like, but in early ovarian cancer detection sensitivity is about 50% and specificity is about 70%. It is hard to say that the marker accuracy is sufficient.

Also, in order to solve the latter, it is important to accurately determine the subtype that carries peritoneal dissemination and lymph node metastasis, and then to formulate an appropriate treatment protocol.
If the predictive diagnosis of ovarian cancer prognosis is performed and it is possible to discriminate the subtype which does not become malignant after carrying out the standard treatment, it is avoided to select excessive treatment for unnecessary patients, and a more aggressive approach is possibly limited to patients with high risk of deteriorating prognosis. Besides benign and malignant, present in tumors arising from the ovaries, there are boundary malignant tumors that follow a process similar to malignancy, although histologically similar benign. These make the select of treatment complicated. Also from this point of view, the marker which is a criterion for accurately determining types of poor prognosis will lead treatment effective.

In recent years, blood circulating DNA (cfDNA) has attracted attention as a marker candidate exceeding CA125. The cfDNA is still insufficient in detection sensitivity, and it requires a better sensitivity marker which can distinguish the specificity ovarian cancer patients with high accuracy (Non-Patent Document 1).
As a marker for predicting ovarian cancer prognosis, by analyzing the DNA methylation profile in ovarian cancer tissue for a specific gene region, a difference was confirmed in the Kaplan-Meier survival curve etc. between the groups of hypermethylation and hypomethylation (Patent Document 1). However, DNA methylation analysis is complicated in terms of the principle of analysis with general diagnostic techniques, and it remains problematic, such as high cost of examination expenses, and the insufficient ability to distinguish between benign tumor and malignant tumor, and false positives. Therefore, further technological breakthroughs are required for technology of the predictive marker for prognosis after onset of cancer.

### REFERENCES

### Patent Documents

Patent Document 1: Patent Publication 2014-525269 Gazette

### Non-Patent Documents

Non-Patent Document 1 : PLos One. 2016 Jun 2; 11(6): e0155495. doi:10.1371/journal. pone. 0155495. Circulating Cell Free DNA as the Diagnostic Marker for Ovarian Cancer: A Systematic Review and Meta-Analysis. Zhou Q1. Li W1. Leng B1, ZhengW1, He Z1, Zuo M1, Chen A1.

### SUMMARY OF THE INVENTION

### Technical Problems of the invention

It is an object of the present invention to provide a technique for determining the degree of malignancy of ovarian cancer and predicting the prognostic process with high accuracy. In addition, the present invention preferably provides a marker technique having accurate discrimination power even in the early stage of clinical stage (stage I).

### Technical Solution

A first aspect of the present invention relates to a diagnostic marker for ovarian cancer prognosis. Specifically, this marker is the marker for predicting ovarian cancer prognosis containing a transcription product of matrix metalloprotease (MMP) 1 gene.

A preferred example of this marker is a predictive marker for ovarian cancer prognosis for evaluating the prognosis of ovarian cancer in a subject using the expression amount of the transcription product of the matrix metalloprotease (MMP) 1 gene in the body fluid sample of the subject.

This marker is used as an element of the test kit for predicting ovarian cancer prognosis. Therefore, the present invention also provides a test kit for predicting ovarian cancer prognosis.

A second aspect of the present invention relates to a method for predicting the ovarian cancer prognosis. This method includes the step of measuring the expression level of a matrix metalloprotease (MMP) 1 gene in a biological sample taken from a subject.

Specifically, the step of measuring the expression level of matrix metalloprotease (MMP) 1 gene is detecting the predictive marker for ovarian cancer prognosis consisting of the transcription product of matrix metalloprotease (MMP) 1 gene to obtain a detection value of the predictive marker for the ovarian cancer prognosis.

Examples of the biological samples are any one or more of ascites of the subject, exosomes recovered from ascites fluid, and tissue samples of the ovarian cancer.

The above method preferably further includes a step of determining whether the detection value of the predictive marker for ovarian cancer prognosis is positive or not. This step is as follows: comparing the detection value of the predictive marker for ovarian cancer prognosis with the detection value of the internal standard gene transcript (example for GAPDH gene) in the biological sample, and determining that the detection value of the predictive marker for ovarian cancer prognosis is positive when the ratio of the detection value of the predictive marker for ovarian cancer prognosis to the detection value of the internal standard gene transcript in the biological sample is the predetermined ratio or more.

An example of the above method is a step of determining whether the detection value of the predictive marker for ovarian cancer prognosis is positive or not, and also includes a step different from above. The step is as follows: determining that the detection value of the predictive marker for ovarian cancer prognosis is positive, when the ratio of the detection value of the predictive marker for ovarian cancer prognosis to the average value of the detection value of the predictive marker for ovarian cancer prognosis in the benign tumor or the ovarian cancer metastasis negative case is a predetermined ratio or more.

### Effect of the Invention

The present invention provides a predictive marker for ovarian cancer prognosis and a kit containing such marker. Furthermore, the present invention also provides a method for predicting the ovarian cancer prognosis.

The malignancy degree of ovarian cancer can be determined by measuring the expression level of MMP1 gene transcription product in ascites and exosomes recovered from ascites, or in ovarian cancer tissue samples. For example, in patients with stage I ovarian cancer, it is possible to diagnose that patients with high expression levels of MMP1 gene transcription product are at high risk of death due to relapse, while patients with low expression levels of MMP1 gene transcription product have a curative course without recurrence. In the current standard therapy, patients with stage I of ovarian cancer have the primary tumor excised and basically end only with surgical therapy. Ovarian cancer cells are known to have a high affinity for mesothelial cells, the main constituent cells of the peritoneum, and are extremely prone to peritoneal metastasis which is one of important prognostic factors. Stage I ovarian cancer refers to a state in which cancer lesions are stopped only in one or both ovaries, and at first glance the 5-year survival rate is also maintained high, but when the disease stage progresses thereafter, there is a tendency to follow the path of malignant progress. As an effect of the present invention, it is possible to determine that expansion surgery (lymph node omentum excision etc.) or a post-treatment (chemotherapy) should be performed according to the MMP1 gene transcript level in an early ovarian cancer patient. With these additional treatments, it is possible to improve patient prognosis for ovarian cancer which is one of the worst prognostic cancers in gynecologic malignant tumors.

### BRIEF DESCRIPTION OF THE DRAWINGS

Figure 1 shows MMP1 gene transcription product in exosomes purified from ascites collected for surgery in patients among three groups of benign tumor, borderline malignant tumor, and malignant tumor.
Figure 2 shows the results of evaluating the performance of the MMP1 gene transcription product as a predictive factor for ovarian cancer prognosis in patients with stage I ovarian cancer.
Figure 3 shows the results of comparing tumor progression in mouse models into which has been orthotopically implanted highly metastatic (ES-2) and non-metastatic (RGM-1) ovarian cancer cell lines.
Figure 4 shows the evaluation results of the influence of A2780 cell line on primary tumor and metastasis promoting action , with exosomes derived from ovarian cancer cell line administered to the mouse model into which has been orthotopically transplanted moderately metastatic ovarian cancer cell line (A 2780).
Figure 5 shows the results of examining the expression levels of the MMP1 gene transcript in cells of highly metastatic (ES-2) and non-metastatic (RMG-1) ovarian cancer cell lines and exosomes derived from these cells. In addition, it shows that these MMP1 gene transcripts are full-length forms.

### DETAILED DESCRIPTION OF THE INVENTION

The first aspect of the invention relates to a predictive marker of ovarian cancer prognosis. Such predictive marker for ovarian cancer prognosis contains transcription product of the matrix metalloprotease (MMP) 1 gene. That is, the transcription product of the MMP1 gene in ovarian cancer patient can be used to accurately determine a group of patients having a high risk of poor prognosis and a group of patients having a low risk of poor prognosis.

Ovarian cancer is classified, for example, epithelial tumor, germ cell tumor, sexual interstitial tumor, metastatic ovarian tumor, and tissue type (serous gonad cancer, clear cell adenocarcinoma, endometrial adenocarcinoma, transitional epithelial cancer, mucinous adenocarcinoma, and mixed type). Also, ovarian cancer is classified as Stages I to IV according to the degree of progression like other cancers. The predictive marker of prognosis in the present invention can identify the risk of malignant of ovarian cancer patients by comparing transcription product of ovarian cancer patients with transcription product of healthy or benign tumor patients.

Most tumors formed in the ovary are considered benign tumors, and there are also ovarian cancers called borderline malignant tumors, which are relatively poorly malignant. And all other tumors are called malignant tumors. Benign tumors basically have no risk of malignization. With the disease proceeding, borderline malignant tumors require the same surgical therapy or chemotherapy as in the case of malignant tumors. When diagnosed as borderline malignant tumor or malignant tumor, the progression phase is established by pathologic findings with laparotomy, and treatment can be determined according to tissue type and the progressive stage. However there is not appropriate prognostic prediction, treatment results of ovarian cancer are lower than those of other cancers.

Prognosis of ovarian cancer is a medical prospect for the course of ovarian cancer. Prognostic predictions are based on any of the followings: the likelihood that the patient will respond to the drug well or poorly, the extent of such response, and the possibility of survival for a certain period of time after surgical removal of the primary cancer and / or chemotherapy. The most appropriate treatment method can be selected for a specific patient by clinically utilizing the method of the present invention, and then the treatment method can be determined. The time to predict the prognosis of ovarian cancer may be either before, during, or after treatment of ovarian cancer.

In the present invention, "long-term survival" means surviving for 3 years or more, preferably 5 years or more, more preferably 8 years or more, most preferably 10 years or more after surgery or other treatments.

The marker is a so-called tumor marker. The marker of the present invention is, for example, a substance which becomes a marker of ovarian cancer cells or exosomes secreted from ovarian cancer cells (marker), and it is a generic name of a substance useful as a criterion for determining of ovarian cancer prognosis and treatment. An example of a substance serving as a marker is an RNA chain which is a transcription product of a gene. The transcription product of a gene means, for example, an RNA strand transcribed with the DNA as a template, that is, an RNA strand synthesized by RNA polymerase, and an RNA strand modified within the cell after transcription. An example of the RNA strand contained in the transcription product is messenger RNA (mRNA). These RNA strands include those processed in cells after transcription.

Examples of transcription product of the matrix metalloprotease (MMP) 1 gene are those having a base sequence amplified using primer pairs of SEQ ID NOS: 3 and 4 or amplified using primer pairs of SEQ ID NOS: 5 and 6.

The present invention also provides a kit comprising a predictive marker for ovarian cancer prognosis. This kit may optionally contain a primer and a nucleic acid probe for the transcription product of the MMP1 gene, which is a prognosis-related gene. The GenBank accession number of the MMP1 gene is NM001145938 and the like. The transcription product of the MMP1 gene can be quantified with the primer sequence, and can be determined by a combination of a forward primer and a reverse primer designed within the same gene sequence region.
In the present specification, the "MMP1 gene" is a gene encoding matrix metalloprotease 1 protein. The MMP1 gene encodes a secreted enzyme which is capable of degrading type I, type II, and type III Interstitial collagen. This gene is part of the MMP gene cluster that sits on human chromosome 11q22.3.

Matrix metalloprotease 1 (MMP 1, another name CLG, CLGN, EC 3.4.24.7) is also well known as a fibroblast collagenase, interstitial collagenase, matrix metallopeptidase-1, or matrix metalloprotease-1 (HGNC: 71551; Entrez Gene: 43122; UniProtKB: P039563; Ensembl: ENSG000001966117; GenBank accession number: NM_0022421).

Examples of forward primer and reverse primer for amplifying cDNA reverse transcribed the transcription product of matrix metalloprotease (MMP) 1 gene are as follows. That is, primer pairs of SEQ ID NOS: 3 and 4, and primer pairs of SEQ ID NOS: 5 and 6.

The predictive kit of ovarian cancer prognosis in the present invention may contain at least a reagent for analyzing gene expression of the marker in the present invention. Then, this kit may contain various equipment, materials, reagents, primers for gene amplification, reagents related to gene amplification, and the like required for carrying out the examination method for ovarian cancer of the present invention.

Examples of such reagent for analyzing gene expression include nucleic acid extract, various enzymes, buffer solution, washing solution, eluate and the like. Specifically, it includes at least an extract solution for suspending a specimen at the time of purifying a nucleic acid, and includes a primer for reverse transcription reaction, a primer for PCR, a reverse transcriptase, a real time PCR Enzymes and so on for detecting the gene transcription product. As a kit element, it may also include a necessary set of equipment such as a microtiter plate capable of simultaneously processing a large number of specimens, a device for DNA amplification, and the like.

The primer contained in the kit is enable to, but not particularly limited to, amplify and detect the transcription product of the MMP1 gene. For example, the size of the primer is at least about 12 bases or more in length, preferably about 15 to 100 bases in length, more preferably about 16 to 50 bases in length, even more preferably about 18 to 35 bases in length. Also, the size of the amplified fragments is different which is optimized according to the type of method for the gene amplification. Therefore, the design of the primer included in the kit is not particularly limited. Such a primer can be prepared by a known method.

The nucleic acid probe contained in the kit is enable to, but not particularly limited to, detect the transcription product of the prognosis-related gene. Nucleic acid probes can be DNA, RNA, modified nucleic acids or chimeric molecules thereof, etc. Considering stability, convenience and the like, DNA probes are preferred, and RNA probes are preferred in consideration of sensitivity. Nucleic acid probes can also be either single stranded or double stranded. The size of the nucleic acid probe is able to, but not particularly limited to, specifically hybridize to the transcription product of the prognosis-related gene, and it is, for example, about 15 or 16 bases or more long, preferably about 15 to 1000 bases long, preferably about 20 to 500 bases long, even more preferably about 20 to 80 bases long. The nucleic acid probe may be provided in a form immobilized on a substrate like a nucleic acid array. Such a nucleic acid probe can be prepared by a method known.

The kit may comprise a means for measuring complexes of MMP1 gene transcription products and reagents. In this case, the reagents and measuring means included in the kit can be provided in isolated form from one another, for example in a different container. The measuring means may be a detection substance labeled with a labeling substance depending on the kind of the reagent. Examples of labeling substances are fluorescent substances such as FITC and FAM, luminescent substances such as luminol, luciferin and lucigenin, radioactive isotopes such as 3 H, 14 C, 32 P, 35 S, and 123 I, and affinities substance such as biotin and streptavidin. The kit may further comprise reverse transcriptase, nucleic acid extract or nucleic acid extraction device. The kit may further comprise a means capable of harvesting a biological sample from an ovarian cancer patient.

Preferably, the predictive marker for ovarian cancer prognosis in the present invention is preferably measured using ascites and exosomes recovered from ascites or ovarian cancer tissue samples.

The predictive marker for ovarian cancer prognosis in the present invention can be used to highly accurately detect a patient having a poor prognosis in a borderline malignant tumor or a malignant tumor patient without showing a false positive to a benign tumor.

When the present invention is applied to a stage I patient, it is possible to accurately determine the prognosis concerning the progression of ovarian cancer also for stage I patients with early cancer.

Next, a method for predicting ovarian cancer prognosis is described. This method is the method of detecting the degree of malignancy of the prognosis of a subject who developed ovarian cancer. This method is to measure the expression level of the MMP1 gene transcription product in a test sample from a subject.

The method for measuring the expression level of the MMP1 gene transcription product can be carried out using a general molecular biological method as well known. Examples of such methods are real-time RT-PCR, microarray analysis, Northern blot analysis, and next generation sequencing.

Next, it can be quantified the expression level of the MMP1 gene transcription product in the ascites and exosomes recovered from the ascites in ovarian cancer patients, or tissue sample of the ovarian cancer. The quantitative method can be accurately determined with a relative value to the expression level of a transcription product such as the GAPDH gene as an internal standard. With using transcription product of the GAPDH gene as an internal standard, when the ratio of the expression level of the MMP1 gene transcription product to the expression level of the GAPDH gene transcription product was a predetermined threshold value or more, it can be determined that a subject of the ovarian cancer has a high risk of poor prognosis. In this case, the reference threshold of the poor prognosis can be set to 0.7%.

### EXAMPLES

The examples were carried out based on the following policy. In order to carry out the present invention, exosomes were prepared from ascites of the subject, and expression level of transcription product of MMP1 gene was measured by real time RT-PCR method. When the ratio of the expression level of the MMP1 gene transcription product to the expression level of the transcription product of the GAPDH gene was higher than the threshold value set at 0.7%, it was determined that a subject of the ovarian cancer had a high risk of poor prognosis.

This threshold value may be changed within a range of 0.5% to 1.5%, for example.

In the benign tumor subjects, the threshold value was 0.7% or less. As the setting basis, according to the expression level of the MMP1 gene transcription product in the ovarian tumor tissue of the stage I ovarian cancer patient, the groups can be classified into the high expression group and the low expression group. With analyzing Kaplan - Meier survival curves of both groups, it is found that a marked reduction in the survival rate was observed in the high expression group, whereas no decrease in the survival rate was observed in the low expression group.

As an example showing that not only the ovarian tumor tissue itself but also the exosome existing in the ascitic fluid can be a test sample for the prognostic evaluation of ovarian cancer, an evaluation was carried out using a mouse. Here, when exosomes derived from high metastatic strains of ovarian cancer cells showing poor prognosis at the time of transplantation were administered to an ovarian cancer peritoneal dissemination model derived from transplanting a moderately metastatic strain into the mice, it is observed promoting metastasis of the abdominal cavity of the ovarian cancer cell line. When exosomes derived from non-metastatic strains of ovarian cancer cells were administered to an ovarian cancer peritoneal dissemination model derived from transplanting a moderately metastatic strain into the mice, it is confirmed that such promotion of metastasis was not observed. In addition, high expression of marked MMP1 gene product was observed in exosomes derived from highly metastatic strains as compared to exosomes derived from non-metastatic strains. It was confirmed that high expression of MMP1 gene product in exosomes was a marker of poor prognosis and used for evaluating the risk of outcome of the peritoneal dissemination.

### Example 1

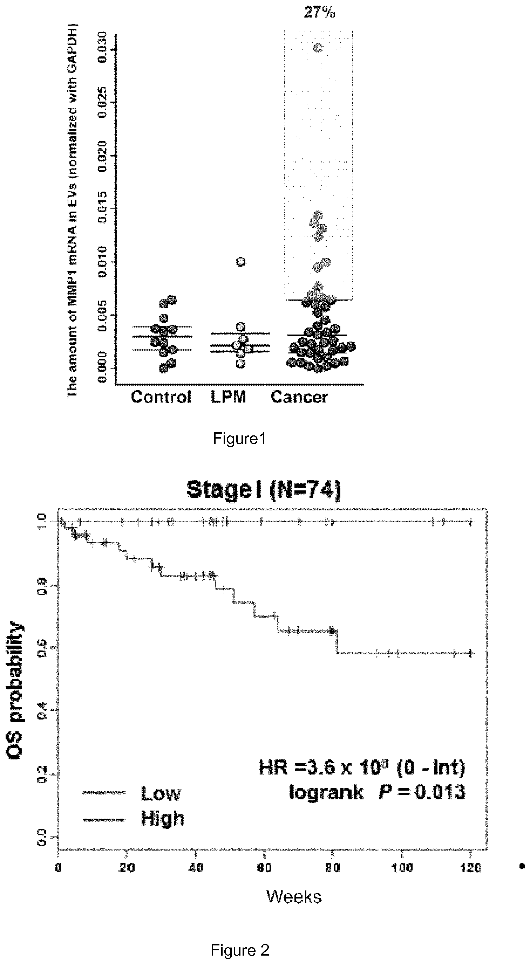

The expression analysis of MMP1 gene transcription product was performed in patients with ovarian cancer of borderline malignant tumor and malignant tumor and in patients having benign tumors in the ovary. The results are shown in Fig. 1.

Separation of exosomes: Ascites collected from surgically removal tumor or abdominal wall puncture in 7 borderline malignant tumors, 41 malignant tumors and 12 benign tumors were treated at 2,000 x g (4 ° C / 10 minutes) for centrifugation. The supernatant fraction except for the precipitate fraction was subjected to a 0.45 µm filter and subjected to centrifugation at 49,000 rpm (4 ° C / 35 minutes) using a TSL-55 rotor (Beckman Coulter Inc.). The precipitate fraction was washed with PBS (-) and centrifuged again at 49,000 rpm (4 ° C / 35 minutes) to obtain an exosome fraction.

Total RNA purification: Total RNA was extracted from exosomes using QIAzol and miRNeasy Mini Kit (Qiagen) according to the instruction manual, and the total RNA concentration was measured.

Real-time RT-PCR: cDNA was synthesized from purified 1 µg of total RNA using High Capacity cDNA Reverse Transcription Kit (Applied Biosystems Inc.). With Plateum SYBR Green qPCR SuperMix UDG (Invitrogen Inc.), Real-Time RT-PCR was performed using a real-time PCR instrument StepOne Real-Time PCR System (Applied Biosystems Inc.). For analysis, normalization was performed with GAPDH gene transcription product. The PCR primers used are as follows.

SEQ ID NO: 1 GAPDH-Fw: gcaccgtcaaggctgagaac
SEQ ID NO: 2 GAPDH-Rv: tggtgaagacgccagtgga
SEQ ID NO: 3 MMP1-Fw: aggtctctgaggtcaagca
SEQ ID NO: 4 MMP1-Rv: ctggttgaaaagcatgagca
PCR reaction conditions were carried out as follows.
50 ° C / 2 min
↓95 ° C / 2 min
↓(95 ° C / 15 sec → 60 ° C / 30 sec) × 40 cycles
Prognostic risk assessment: The ratio of the expression level of transcription product of the MMP1 gene to the expression level of transcription product of the GAPDH gene was calculated. A plot of the expression ratio for each of the borderline malignant tumor group (LPM), the malignant tumor group (Cancer), and the benign tumor group (Control) was depicted. It shows that the MMP1 gene transcription products in the object group surrounded by the orange color of the malignant tumor group (Cancer) were highly expressed comparing with those in the benign tumor group (Control). The proportion was 27% of the total malignant tumor group (Cancer). Based on these results, the subjects can be determined highly expressing these MMP1 gene transcription product if the expression ratio of the MMP1 gene transcription product to the GAPDH gene transcription product in these subjects is 0.7% or more which is the reference value for determining, and these subjects can be classified to have a risk of poor prognosis for ovarian cancer (Fig. 1).

### Example 2

In a Kaplan Meier plotter, it shows a public microarray dataset providing analysis data of the gene expression in various cancer clinical specimens. 74 patients of Stage I ovarian cancer were selected from 1582 ovarian cancer patients, and Kaplan Meier survival curve analysis of MMP1 gene transcription product was performed (MMP1 probe: 204475_at). The results are shown in Fig. 2. In patients of Stage I ovarian cancer, high expression of MMP1 gene in tumor tissue was found to be highly correlated with subsequent prognosis deterioration (hazard ratio: 3.6 × 10⁸, log rank test: p = 0.014).

### Example 3

RMG-1 and ES-2 of a human ovarian cancer cell line into which a luciferase gene had been introduced were grafted orthotopically into the left ovary of CB17 / icr-scid / scidJcl mouse, and were imaged in vivo using an IVIS system until a maximum of 8 weeks. As a result, the proliferative and invasive properties of non-metastatic strain RMG-1 and highly metastatic strain ES-2 in the mice were evaluated.

Cell preparation: The RMG-1 cell line was cultured in DMEM / F12 medium supplemented with 10% inactivated FBS and 1% antibiotic-antimycotic solution (Invitrogen Inc.), and the ES-2 cell line was cultured in McCoy's 5A medium containing 10% inactivated FBS and 1% antibiotic-antimycotic solution (Invitrogen Inc.). Culture conditions were set at 37 ° C / 5% CO₂ concentration. Both cells were modified so as to express the luciferase protein by introducing the luciferase gene.

Cell transplantation: The prepared RMG-1 cell line and ES-2 cell line were orthotopically transplanted to the left ovary of CB17 / icr-scid / scidJcl mouse which is an immunodeficient mouse lacking T cell and B cell. The number of transplanted cells was transplanted at 1 × 10⁶ cells.

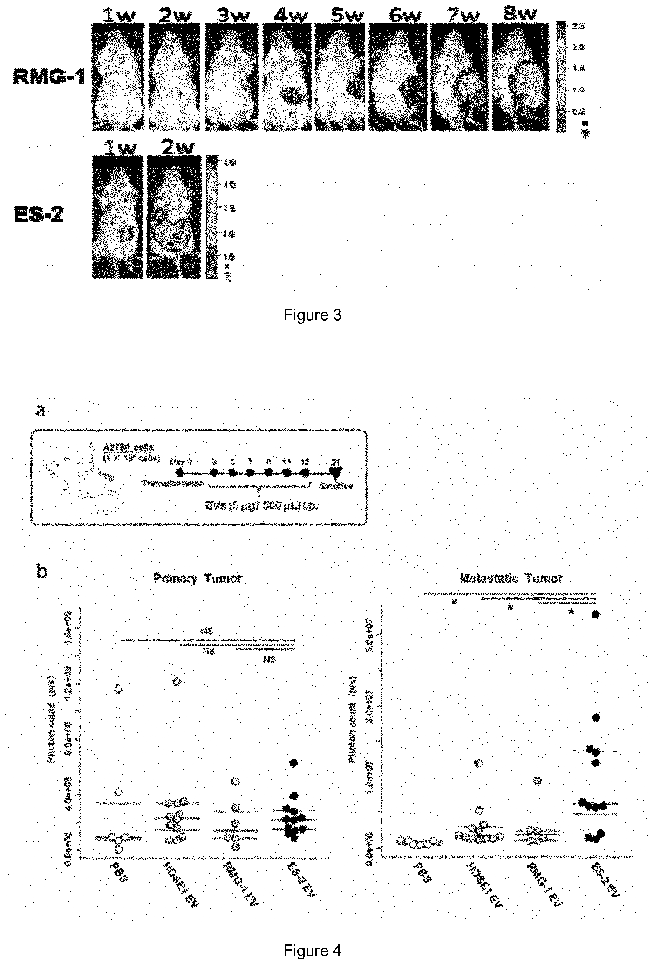

In vivo imaging: The proliferation and infiltration of cancer cells were evaluated by detecting the luminescence of luciferase using an IVIS Spectrum imaging system (Caliper Life Science) from 1 week after ovarian cancer cell line transplantation. For detection of luciferase activity, D-luciferin was intraperitoneally administered at 150 mg / kg, and light emission of the whole body of the mouse was detected after about 10 minutes. The image analysis was performed with LIVING IMAGE 4.4 software (Caliper Life Science). The results are shown in Fig. 3. As shown in Fig. 3, in the highly metastatic strain ES-2, primary tumor and metastatic invasion into the peritoneal cavity were observed two weeks after the cell transplantation, whereas in the non-metastatic strain RMG-1, only the increase in the primary tumor was observed during observation period of 8 weeks. This confirmed that RGM-1 is a non-metastatic, low-grade ovarian cancer cell line, while ES-2 is a highly metastatic high-grade ovarian cancer cell line.

### Example 4

It was evaluated that the effect of exosomes derived from ovarian cancer cell lines having different degrees of metastasis malignancy and human ovarian surface epithelial cells on metastatic invasion of ovarian cancer cells.

Cell preparation: The A2780 cell line was cultured in RPMI-1640 medium supplemented with 10% inactivated FBS and 1% antibiotic-antimycotic solution (Invitrogen Inc.). Culture conditions were set at 37 ° C / 5% CO₂ concentration. The A2780 cell line was modified to express the luciferase protein by introducing the luciferase gene.

Preparation of exosomes: Human ovary surface epithelial cells (HOSE 1) and RGM-1 were cultured in DMEM / F 12 medium supplemented with 10% inactivated FBS and 1% antibiotic-antimycotic solution (Invitrogen Inc.), and ES-2 cells Strains were cultured in McCoy's 5 A medium supplemented with 10% inactivated FBS and 1% antibiotic-antimycotic solution (Invitrogen Inc.). Culture conditions were set at 37 ° C / 5% CO₂ concentration. When the cells reached about 90% proliferation, the cells were washed with PBS (-) and replaced with serum-free medium. Advanced DMEM medium was used for ES-2, and advanced DMEM / F12 was used for RGM-1 and HOSE 1. After 48 hours of culture, the culture supernatant was recovered. The culture supernatant was subjected to centrifugation at 2,000 × g (4 ° C/10 min) to remove cellular residues and the like, and the supernatant fraction was applied to a 0.22 µm filter (Millipore Inc.) for purification. The supernatant fraction was subjected to centrifugation at 35,000 rpm (4 ° C/70 minutes) using a SW 41 Ti rotor (Beckman Coulter Inc.). The precipitated fraction was washed with PSB (-) and centrifuged again at 35,000 rpm (4 ° / 70 min) to obtain an exosome fraction.

Production of Model Mouse: The prepared A2780 cell line was grafted orthotopically to the left ovary of CB17 / icr-scid / scidJcl mouse, which is an immunodeficient mouse lacking T cells and B cells. The number of transplanted cells was transplanted at 1 × 10⁶ cells. 5 µg of exosomes prepared from ES-2, RGM-1, HOSE 1 were administered intraperitoneally to the mouse. The time and frequency of administration were set daily from 3 days to 13 days after the A 2780 cell transplantation (total 6 doses: total exosome amount 30 µg) (Fig. 4 a).

Evaluation of metastasis: The primary tumor and metastatic tumor were evaluated 21 days after A2780 cell transplantation. Specifically, proliferation and infiltration of cancer cells were evaluated by detecting the luminescence of luciferase in the A2780 cell line using the IVIS Spectrum imaging system (Caliper Life Science Inc.). For detection of luciferase activity, D-luciferin was intraperitoneally administered at 150 mg / kg, and about 10 minutes later, luminescence in the whole body of the mouse was detected. In the detection, the whole body of the mouse, the excised primary tumor, and the peritoneal cavity after removal of the primary tumor were examined. The image analysis was performed with LIVING IMAGE 4.4 software (Caliper Life Science Inc.). The results are shown in Fig. 4b. For the group which is administered exosome derived from HOSE 1 and the group which is administered exosome derived from RMG-1, the metastasis to the peritoneal cavity was almost the same as the median value of the group administered with PBS (-), whereas in the group which is administered exosome derived from ES-2, the metastasis to the peritoneal cavity was markedly promoted. On the other hand, no significant difference was detected in tumor growth of the primary tumor among the group which is administered exosome derived from HOSE 1, the group which is administered exosome derived from RMG-1, and the group which is administered exosome derived from ES-2.

### Example 5

A comparison was made between the expression levels of MMP1 gene transcription product contained in ovarian cancer cell lines and exosome fractions derived from these ovarian cancer cell lines.

Cell preparation: Human ovary surface epithelial cells (HOSE 1) and RGM-1 were cultured in DMEM / F12 medium supplemented with 10% inactivated FBS and 1% antibiotic-antimycotic solution (Invitrogen Inc.), and ES-2 cells Strains were cultured in McCoy's 5 A medium supplemented with 10% inactivated FBS and 1% antibiotic-antimycotic solution (Invitrogen Inc.). Culture conditions were set at 37°C / 5%CO₂ concentration.

Preparation of exosomes: When the cells reached about 90% proliferation, the cells were washed with PBS (-) and replaced with serum-free medium. The advanced DMEM medium was used for ES-2, and advanced DMEM / F12 was used for RGM-1 and HOSE 1. After 48 hours of culture, the culture supernatant was recovered. The culture supernatant was subjected to centrifugation at 2,000 × g (4°C/10 min) to remove cellular residues and the like, and the supernatant fraction was applied to a 0.22 µm filter (Millipore Inc.) for purification. The supernatant fraction was subjected to centrifugation at 35,000 rpm (4°C/70 minutes) using a SW 41 Ti rotor (Beckman Coulter Inc.). The precipitated fraction was washed with PSB (-) and centrifuged again at 35,000 rpm (4 ° C / 70 minutes) to obtain an exosome fraction.

Total RNA purification: Total RNA was extracted from exosomes using QIAzol and miRNeasy Mini Kit (Qiagen) according to the instruction manual, and the total RNA concentration was measured.

Real-time RT-PCR: cDNA was synthesized from purified 1 µg of total RNA using High Capacity cDNA Reverse Transcription Kit (Applied Biosystems Inc.). With Plateum SYBR Green qPCR SuperMix UDG (Invitrogen Inc.), Real-time RT-PCR was performed using real PCR equipment StepOne Real-Time PCR System (Applied Biosystems Inc.). For analysis, normalization was performed with GAPDH gene transcription product. The PCR primers used were as follows.

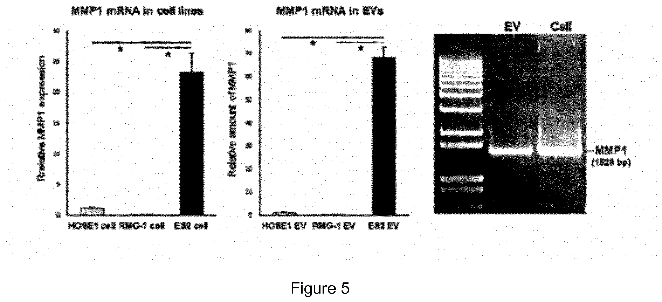

SEQ ID NO: 1 GAPDH-Fw: gcaccgtcaaggctgagaac
SEQ ID NO: 2 GAPDH-Rv: tggtgaagacgccagtgga
SEQ ID NO: 3 MMP1-Fw: aggtctctgaggtcaagca
SEQ ID NO: 4 MMP1-Rv: ctggttgaaaagcatgagca
PCR reaction conditions were carried out as follows.
50 ° C / 2 min
↓95 ° C / 2 min
↓(95 ° C / 15 sec → 60 ° C / 30 sec) × 40
Quantitative Results: As a result of comparing the expression levels of MMP1 gene transcription products, the expression level of MMP1 gene transcription product in cells and exosomes derived from the highly metastatic ovarian cancer cell line ES2 was higher than those in RMG-1 and HOSE1 about 22 times and about 65 times, respectively (Fig. 5).

Confirmation of MMP1 gene transcription product: In order to verify that transcription product of MMP1 gene contained in the exosome was the full-length type, cDNA was synthesized by SuperScript III First-Strand Synthesis System for RT-PCR (Invitrogen Inc.) from the total RNA according to the manufacturer's instructions. Using the synthesized cDNA as a template, a PCR reaction was carried out using Takara Ex Taq (Takara Bio Inc.). The PCR primers used are as follows, and the DNA fragment to be amplified is 1,528 base pairs.

SEQ ID NO: 5 MMP1-Full-Fw: gatattggagcagcaagagg
SEQ ID NO: 6 MMP1-Full-Rv: caccttctttggactcacac
PCR reaction conditions were carried out as follows.
(98 ° C / 10 sec → 60 ° C / 30 sec → 72 ° C / 2 min) × 30 cycles
As a result, it was revealed that the ES2-derived exosome contains transcription product of the full-length MMP1 gene.

Summarizing the essence of the above examples, Example 2 shows that the MMP1 gene transcription product is highly expressed in the ovarian tumor tissue of the poor prognosis group of ovarian cancer in Stage I, and also shows that exosome derived from ascites in the patient with ovarian cancer malignant tumor has high expression of MMP1 gene transcription product which is detected in about 27% of patients and not observed in benign tumor patients. Furthermore, exosomes derived from ES 2, an ovarian cancer cell line with highly metastatic to the peritoneal cavity shown in Example 3, has been shown to have the function of promoting the metastasis to peritoneal cavity of A 2780, an ovarian cancer cell line with moderate metastatic, and such function has not observed by exosomes derived from RMG - 1, an ovarian cancer cell line with non-metastatic to the peritoneal cavity. In Example 5, it was found that the MMP1 gene transcription product is extremely highly expressed in the exosome derived from ES-2 as compared with the exosome derived from RMG-1. Combining these complex insights, the MMP1 gene transcription product in the ascites from subjects with ovarian cancer and exosome recovered from ascites, or in ovarian cancer tissue samples, can be used as an accurate prognostic marker for an ovarian cancer patient.

### Possibility for business

The present invention relates to the prognostic risk assessment after onset of ovarian cancer, and it is possible to detect a patient presumed to have a poor prognosis by an examination using a marker according to the present invention and the like. In ovarian cancer diagnosis, collection of ascites is a necessary examination normally performed, and it is easy to obtain ascites or exosomes in ascites using biological samples generated from existing medical treatments without new invasive sampling. Therefore, it does not increase the burden on the patient, and it is an examination easy to accept even in the medical field. The information obtained therefrom can be important for determining subsequent aggressive treatment interventions in order to improve outcomes of patients assumed to have poor prognosis. Therefore, the present invention can be effectively utilized in the medical examination industry, the medical device industry, the medical analysis industry, the medical industry in the cancer treatment field, and the like.

### Sequence table Free text

SEQ ID NO:1; A PCR primer specific to transcriptional product of GAPDH gene.
SEQ ID NO: 2; A PCR primer specific to transcriptional product of GAPDH gene.
SEQ ID NO:3; A PCR primer specific to transcriptional product of MMP-1 gene.
SEQ ID NO:4; A PCR primer specific to transcriptional product of MMP-1 gene.
SEQ ID NO:5; A PCR primer specific to transcriptional product of MMP-1 gene.
SEQ ID NO:6; A PCR primer specific to transcriptional product of MMP-1 gene.

## Claims

1. A method for predicting the prognosis of ovarian cancer, comprising obtaining a detection value of a transcription product of full-length matrix metalloprotease (MMP) 1 gene in ascites collected from a subject or exosomes recovered from ascites, and measuring the expression level of the full-length matrix metalloprotease (MMP) 1 gene.

2. The method of claim 1, further comprising the step of comparing the detection value of the transcription product of the full-length matrix metalloprotease (MMP) 1 gene with a detection value of a transcription product of the internal standard gene in the ascites collected from the subject or the exosomes recovered from the ascites, and determining that the detection value of the transcription product of the full-length matrix metalloprotease (MMP) 1 gene is positive when the ratio of the detection value of the transcription product of the full-length matrix metalloprotease (MMP) 1 gene to the detection value of the transcription product of the internal standard gene in the ascites or the exosomes recovered from the ascites is a predetermined ratio or more.


SEQUENCE LISTING

<110>  BIOMIMETICS SYMPATHIES Inc.
       National Cancer Center

<120>  Transcript product of MMP-1 gene as a prognostic marker for
       ovarian cancer and diagnostic methods

<130>  16-135P

<160>  6

<170>  PatentIn version 3.5

<210>  1
<211>  20
<212>  DNA
<213>  Artificial Sequence

<220>
<223>  primer

<400>  1
gcaccgtcaa ggctgagaac                                                  20


<210>  2
<211>  19
<212>  DNA
<213>  Artificial Sequence

<220>
<223>  primer

<400>  2
tggtgaagac gccagtgga                                                   19


<210>  3
<211>  20
<212>  DNA
<213>  Artificial Sequence

<220>
<223>  primer

<400>  3
aggtctctga gggtcaagca                                                  20


<210>  4
<211>  20
<212>  DNA
<213>  Artificial Sequence

<220>
<223>  primer

<400>  4
ctggttgaaa agcatgagca                                                  20


<210>  5

<211>  20
<212>  DNA
<213>  Artificial Sequence

<220>
<223>  primer

<400>  5
gatattggag cagcaagagg                                                    20


<210>  6
<211>  20
<212>  DNA
<213>  Artificial Sequence

<220>
<223>  primer

<400>  6
caccttcttt ggactcacac                                                    20